Europäisches Patentamt

European Patent Office

Office européen des brevets

(19)

(11) Veröffentlichungsnummer: **0 162 211**
**B1**

(12) **EUROPÄISCHE PATENTSCHRIFT**

(45) Veröffentlichungstag der Patentschrift :
30.08.89

(51) Int. Cl.⁴: $A\ 61\ K\ 6/10$, $C\ 08\ L\ 83/07$

(21) Anmeldenummer : 85102801.9

(22) Anmeldetag : 12.03.85

(54) Dimensionsstabile Abformmassen.

(30) Priorität : 23.03.84 DE 3410646

(43) Veröffentlichungstag der Anmeldung :
27.11.85 Patentblatt 85/48

(45) Bekanntmachung des Hinweises auf die Patenterteilung : 30.08.89 Patentblatt 89/35

(84) Benannte Vertragsstaaten :
AT BE CH DE FR GB IT LI LU NL SE

(56) Entgegenhaltungen :
DE—A— 2 926 405
FR—A— 2 400 052
US—A— 2 709 161
US—A— 4 356 116
US—A— 4 430 461

(73) Patentinhaber : BAYER AG

D-5090 Leverkusen 1 Bayerwerk (DE)

(72) Erfinder : Schwabe, Peter, Dr.
Dudweiler Strasse 17
D-5090 Leverkusen 1 (DE)
Erfinder : Schlak, Ottfried, Dr.
Carl-Duisberg-Strasse 331
D-5090 Leverkusen 1 (DE)
Erfinder : Knispel, Gottfried, Dr.
Richard-Wagner-Strasse 3
D-5090 Leverkusen 1 (DE)
Erfinder : Voigt, Reiner
Gustav-Radbruch-Strasse 14
D-5090 Leverkusen 1 (DE)

EP 0 162 211 B1

**Beschreibung**

Die vorliegende Erfindung betrifft dimensionsstabile Abform- bzw. Dubliermassen auf Polysiloxanbasis, die insbesondere im Dentalbereich Anwendung finden.

Die vorliegende Erfindung betrifft insbesondere additionsvernetzende Vinylsilikon-Pasten zum Herstellen genauer Abformungen bezahnter, teilbezahnter und unbezahnter Kiefer sowie von Gipsmodellen. Hierbei handelt es sich um ein kaltvulkanisierendes Zweikomponenten-Silikonkautschuksystem, bei dem die vernetzerhaltige Basispaste mit einer Katalysatorpaste vermengt wird und bei Raumtemperatur vernetzt.

Derartige Systeme sind an sich bekannt (vgl. z. B. R.G. Craig, Restorative Dental Materials, The C.V. Mosby-Comp., St. Louis, 1980, S. 195 ff).

In der Zahntechnik werden solche Abformmassen zur Herstellung von Gipsmodellen und zur Vervielfältigung dieser Gipsmodelle eingesetzt. Am gebräuchlichsten sind Dubliermassen auf Basis von Agar Agar, die allerdings den Nachteil einer genauen Temperaturführung und somit eine langsame Weiterverarbeitbarkeit aufweisen. Solche Massen bedingen eine begrenzte Lagerfähigkeit der Abformung infolge langsamen Verdunstens des Wassers.

Auf dem Sektor der Abformmassen sind die Silikon-Pasten weit verbreitet. Im allgemeinen bestehen sie aus einem mit Füllstoffen vermischten Silikonöl auf Basis hydroxylendgestoppten Polydimethylsiloxans — wegen vieler Applikationsmethoden in verschiedenen Konsistenzen angeboten — und einer flüssigen oder pastenförmigen Härterkomponente, die ein Metallsalz einer (Mono)carbonsäure als Katalysator und einen Kieselsäureester als Vernetzer enthält (vgl. z. B. W. Noll, Chemie u. Technologie der Silicone, Verlag Chemie, Weinheim 2. Aufl. 1964, S. 339/40).

Die beiden Komponenten des Silikonsystems werden vor der Anwendung gemischt und vernetzen dann bei Raumtemperatur innerhalb von 2-5 Minuten infolge einer Polykondensationsreaktion. Hierbei entstehen neben dem vernetzten Silikongummi noch geringe Mengen Alkohol, die langsam aus dem Gummi herausdiffundieren und eine lineare Schrumpfung hervorrufen. Bei den Abformungen führt sie zur Dimensionsänderung und damit zu Ungenauigkeiten.

Wesentlich geringer ist die Schrumpfung bei den seit einigen Jahren bekannten Vinylsilikon-Abformungen, die durch Polyadditionsreaktion vernetzen. Diese Massen bestehen aus einer Silikonöl, Füllstoff und Vernetzer enthaltenden Basispaste und einer Katalysatorpaste aus Silikonöl, Füllstoff und Katalysator.

Bei dem Silikonöl handelt es sich überwiegend um ein vinylendgestopptes Polydimethylsiloxan, der Vernetzer enthält reaktive SiH-Gruppen und der Katalysator besteht aus Platin bzw. einem Platinkomplex. Zu der größeren Dimensionsgenauigkeit des Modells kommt bei diesem System noch die bessere Dosierbarkeit von Basis- und Katalysatorpaste infolge gleicher Pastenviskosität und dem abgestimmten Mischungsverhältnis von 1 :1 der beiden Pasten hinzu, sowie die völlige Geschmacks- und Geruchlosigkeit der Pasten.

Die Ausgangssubstanzen wie vinylgruppenhaltige Silikonöle, trimethylsiloxyendgestoppte Polydimethylsiloxane und SiH-gruppenhaltige Polysiloxane (Vernetzersubstanzen) werden in an sich bekannter Weise hergestellt (vgl. z. B. W. Noll, l.c. S. 162-206).

Wird bei diesen Ausgangsmaterialien nach Standardmethoden (z. B. durch DIN 51 581) der Gehalt an flüchtigen Oligomeren bestimmt, stellt man in der Regel einen Gewichtsverlust von etwa 1 bis 2 Gew.- % fest. Stellt man mit derartigen Rohstoffen Abform- bzw. Dubliermassen her, erhält man nur unbefriedigende Ergebnisse. Mißt man z. B. die Dimensionsänderung (gemäß ADA-Spezifikation Nr. 19) nach 24 Std. so stellt man je nach Füllgrad der Pasten einen linearen Schwund von 0,25-0,45 % fest. Diese Werte sind niedriger als die von den Abformmassen auf Basis kondensationsvernetzender Silikone, welche bei 0,5-0,9 % liegen. Trotzdem sollten die Werte unter 0,2 % liegen, um die optimale Paßgenauigkeit des späteren Zahnersatzes zu gewährleisten.

Überraschenderweise wurde nun gefunden, daß es möglich ist, diese Werte zu erzielen, wenn man erfindungsgemäß die flüssigen Komponenten in den Massen wie die vinyl- und trimethyl-endgestoppten Polydimethylsiloxane sowie die SiH-gruppenhaltigen Vernetzer nach bekannten Verfahren, z. B. mit einem Dünnschicht-Verdampfer oder einem Fallfilmverdampfer derart ausheizt, daß sie nach der obigen Standardmethode einen Gehalt von weniger als 1,5 Gewichtsprozent, bevorzugt von 0-0,8 Gew.- % an flüchtigen Oligomeren aufweisen.

Gegenstand der vorliegenden Erfindung sind somit dimensionsstabile, bei Umgebungstemperatur additionsvernetzende Abform- und Dubliermassen auf Polysiloxanbasis enthaltend

a) Organopolysiloxane mit zwei oder mehr Vinylgruppen im Molekül,
b) gegebenenfalls Organopolysiloxane ohne reaktive Gruppen,
c) Organohydrogenpolysiloxane mit zwei oder mehr Si-H-Gruppen im Molekül,
d) Katalysator,
e) Füllstoffe sowie gegebenenfalls weitere übliche Zusatz-, Hilfs- und Farbstoffe

welche dadurch gekennzeichnet sind, daß die flüssigen Polysiloxan-Komponenten a)-c) einen Gehalt an flüchtigen Oligomeren von maximal 1,5 Gew.- % aufweisen.

So zeichnen sich die erfindungsgemäßen Vinylsilikonpasten einmal für die Herstellung genauer Abformungen von bezahnten, teilbezahnten und unbezahnten Kiefern durch ihre geringe Dimensionsänderung von ≤0,2 % nach einer Lagerung von 24 Stunden bei 23 °C, gemessen nach der ADA-Spezifikation Nr. 19, aus. Die gleichen geringen Werte werden auch von erfindungsgemäßen Vinylsilikonpasten erzielt, die zum Dublieren von Gipsmodellen verwendet werden.

Als Ausgangsstoffe für den Gegenstand der vorliegenden Erfindung sind folgende Materialien geeignet :

Bei dem Silikonöl (a) handelt es sich um ein Polydimethylsiloxan mit ungesättigten Kohlenwasserstoff-Gruppen, vorzugsweise Vinylgruppen an mindestens zwei Silicumatomen, dessen Viskosität im Bereich von 500 bis 200 000 mPa.s bei 20 °C liegen kann, je nach gewünschter Viskosität der formulierten Pasten.

Vinylsilikonöle sind erfindungsgemäß durch Passage einer Dünnschichtverdampferanlage auf einen Gehalt an flüchtigen Oligomeren von max. 1,5 Gew.- %, vorzugsweise 0-0,8 Gew.- % eingestellt worden.

Die Silikonöle (b) sind trimethylsiloxy-endgestoppte Polydimethylsiloxane mit einer Viskosität von 50 bis 2 000 mPa.s bei 20 °C, welche erfindungsgemäß nach Passage einer Dünnschichtverdampferanlage einen Gehalt an flüchtigen Oligomeren von max. 1,5 Gew.- % aufweisen.

Diese Silikonöle mit einem Anteil von bis 40 Gew.- %, bezogen auf die Gesamtmenge Polydimethylsiloxan, dienen vorzugsweise als Weichmacher in den Dubliermassen.

Der Vernetzer (c) ist ein Polydimethylsiloxan, welches in seinem Molekül Wasserstoffatome an mindestens zwei Siliziumatomen und erfindungsgemäß einen Gehalt an flüchtigen Oligomeren von max. 1,5 Gew.- % nach Passage einer Dünnschichtverdampferanlage aufweist.

Bei dem Katalysator (d) handelt es sich bevorzugt um einen Platinkomplex, der aus Hexachloroplatin-(IV)-säure hergestellt wurde. Auch diese Verbindungen sind an sich bekannt. Geeignet sind auch andere Platinverbindungen, die die Additionsvernetzungsreaktion beschleunigen. Gut geeignet sind z. B. Platin-Siloxan-Komplexe, wie sie z. B. in US-PS 3 715 334, US-PS 3 775 352 und US-PS 3 814 730 beschrieben sind.

Unter den Füllstoffen (e) versteht man Quarz- und Cristobalitmehle, Calciumsulfat, Calciumcarbonat, Diatomeenerde, gefälltes und pyrogen hergestelltes Siliciumdioxid mit unbeladener und beladener Oberfläche.

Farbstoffe werden zur Unterscheidung der Basis- und Katalysatorpaste und zur Mischkontrolle eingesetzt. Anorganische und organische Farbpigmente werden üblicherweise eingesetzt.

Anhand der folgenden Beispiele soll die vorliegende Erfindung noch näher erläutert werden.

## Beispiel 1 (Vergleich)

Der Gehalt an flüchtigen Oligomeren in den vinyl- und trimethylsiloxy endgestoppten Polydimethylsiloxanen und SiH-gruppenhaltigen Vernetzern wird mittels einer Standardmethode durch Ausheizen einer Probe von 200-400 mg in einer Trockenpistole bei 145 °C und 20-30 mbar während 45 Minuten bestimmt. Bei den Füllstoffen wird der Trocknungsverlust nach einer Lagerung bei 110 °C während 1 Stunde ermittelt.

Eine Basispaste wurde hergestellt durch Vermischen in einem Kneter von 440 Teilen vinylendgestopptem Polydimethylsiloxan mit einer Viskosität von 10 000 mPa.s bei 20 °C und einem Gehalt an flüchtigen Oligomeren von 1,9 Gew.- %, 50 Teilen SiH-Gruppen-haltigen Polydimethylsiloxan mit einer Viskosität von 50 mPa.s bei 20 °C und einem Gehalt von 1,7 Gew.- % flüchtiger Oligomere, 475 Teilen Quarzfeinstmehl mit einem Trocknungsverlust von 0,35 Gew.- %, 30 Teilen pyrogen hergestellte und oberflächlich behandelte Kieselsäure mit der speziellen Oberfläche von 50 m²/g und 0,65 Gew.- % Trocknungsverlust und 5 Teile anorganischem Farbpigment.

Die Herstellung einer Katalysatorpaste erfolgte durch Vermischen in einem Kneter von 485 Teilen vinylendgestopptem Polydimethylsiloxan mit einer Viskosität von 10 000 mPa.s bei 20 °C und einem Gehalt von 1,9 Gew.- % flüchtiger Oligomerer, 485 Teile Quarzfeinstmehl mit 0,35 Gew.- % Trocknungsverlust, 29,8 Teilen pyrogen hergestellte und oberflächlich behandelte Kieselsäure mit einer speziellen Oberfläche von 50 m²/g und 0,65 Gew.- % Trocknungsverlust und 0,2 Teilen Platin-Siloxan-Komplex.

## Beispiel 2

Nach den Angaben in Beispiel 1 wurde eine Basis- und eine Katalysatorpaste hergestellt mit der Änderung, daß das vinylendgestoppte Polydimethylsiloxan einen Oligomerengehalt von 0,55 Gew.- % und der Vernetzer einen von 0,45 Gew.- % aufweist. Wie aus den Angaben nach Beispiel 7 ersichtlich ist, wurde eine dimensionsstabile Abformmasse erhalten.

## Beispiel 3 (Vergleich)

In einem Kneter wurde eine weitere Basispaste hergestellt durch Vermischen von 180 Teilen vinylendgestopptem Polydimethylsiloxan mit einer Viskosität von 10 000 mPa.s bei 20 °C und einem Oligomerengehalt von 1,9 Gew.- %, 300 Teilen vinylendgestopptem Polydimethylsiloxan mit einer Viskosität von 1 000 mPa.s und 1,75 Gew.- % Oligomeren, 300 Teilen SiH-Gruppen-haltigen Polydimethylsiloxan mit einer Viskosität von 95 mPa.s bei 20 °C und 1,95 Gew.- % Oligomeren, 210 Teilen gefällter und oberflächenbehandelter Kieselsäure mit einer speziellen Oberfläche von 90 m²/g und 1,2 Gew.- % Trocknungsverlust und 10 Teilen anorganischem Farbpigment.

Eine Katalysatorpaste wurde in einem Kneter hergestellt durch Vermischen von 78 Teilen vinylendgestoppten Polydimethylsiloxan mit einer Viskosität von 10 000 mPa.s bei 20 °C und 1,9 Gew.- % Oligomeren, 710 Teilen vinylendgestopptem Polydimethylsiloxan mit einer Viskosität von 1 000 mPa.s bei 20 °C und 1,75 Gew.- % Oligomeren, 210 Teilen gefällte und oberflächenbehandelte Kieselsäure mit einer speziellen Oberfläche von 90 m²/g und 1,2 Gew.- % Trocknungsverlust, 1,8 Teilen anorganischem Farbpigment und 0,2 Teilen Platin-Siloxan-Komplex.

## Beispiel 4

Nach den Angaben in Beispiel 3 wurde eine Basis- und Katalysatorpaste durch Vermischen im Kneter hergestellt. Das vinylendgestoppte Polydimethylsiloxan mit einer Viskosität von 10 000 mPa.s wies einen Oligomerengehalt von 0,55 Gew.- %, das vinylendgestoppte Polydimethylsiloxan mit einer Viskosität von 1 000 mPa.s einen von 0,4 Gew.- % und der Vernetzer einen von 0,45 Gew.- % auf. Die Angaben über die Dimensionsstabilität sind nach Beispiel 7 aufgeführt.

## Beispiel 5 (Vergleich)

Eine Basispaste wurde hergestellt durch Vermischen in einem Kneter von 550 Teilen vinylendgestopptem Polydimethylsiloxan mit einer Viskosität von 10 000 mPa.s bei 20 °C und einem Oligomerengehalt von 1,9 Gew.- %, 200 Teilen trimethylsilyl-endgestopptem Polydimethylsiloxan mit einer Viskosität von 120 mPa.s und 1,65 Gew.- % Oligomeren, 200 Teilen SiH-Gruppen-haltigen Polydimethylsiloxan mit einer Viskosität von 95 mPa.s bei 20 °C und 1,95 Gew.- % Oligomeren und 50 Teilen gefällter und oberflächenbehandelter Kieselsäure mit einer speziellen Oberfläche von 90 m²/g und 1,2 Gew.- % Trockenverlust.

In einem Kneter wurde die Katalysatorpaste hergestellt durch Vermischen von 540 Teilen vinylendgestopptem Polydimethylsiloxan mit einer Viskosität von 10 000 mPa.s bei 20 °C und einem Oligomerengehalt von 1,9 Gew.- %, 400 Teilen trimethylsilyl-endgestopptem Polydimethylsiloxan mit einer Viskosität von 120 mPa.s und 1,65 Gew.- % Oligomeren, 50 Teilen gefällter und oberflächenbehandelter Kieselsäure mit einer speziellen Oberfläche von 90 m²/g und 1,2 Gew.- % Trockenverlust, 9,8 Teilen anorganischem Farbpigment und 0,2 Teile Platin-Siloxan-Komplex.

## Beispiel 6

Nach den Angaben in Beispiel 5 wurde eine Basis- und Katalysatorpaste durch Vermischen im Kneter hergestellt. Das vinylendgestoppte Polydimethylsiloxan mit einer Viskosität von 10 000 mPa.s wies einen Oligomerengehalt von 0,55 Gew.- %, das trimethylsilyl-endgestoppte Polydimethylsiloxan mit einer Viskosität von 120 mPa.s einen von 0,25 Gew.- % und der Vernetzer einen von 0,45 Gew.- % auf. Wie aus der Angabe nach Beispiel 7 ersichtlich ist, wurde eine dimensionsstabile Dubliermasse erhalten.

## Beispiel 7

Entsprechend der Spezifikation Nr. 19 der American Dental Association (ADA) wurden jeweils Basis- und Katalysatorpaste gewichtsmäßig 1 : 1 (Beispiel 1-4) bzw. 9 : 1 (Beispiel 5-6) gemischt und 90 Sekunden nach Mischbeginn auf einen mit Rillen versehenen Block in einer Form gegeben. Die Form wird erst mit einer Polyethylenfolie und dann mit einer starren, flachen Metallplatte abgedeckt und unter ausreichender Krafteinwirkung fest gegen die Form gedrückt. Dieser Zusammenbau wird sofort in ein Wasserbad von 32 ± 1 °C Temperatur gestellt. Nach 7,5 Minuten (Beispiel 1-4) bzw. 10 Minuten (Beispiel 5-6) wird der Zusammenbau aus dem Wasserbad genommen und Form und Testblock werden voneinander getrennt, sowie die Abformung mittels eines Hebers aus der Form gedrückt. Die Seite gegenüber der Bezugsmarkierung wird mit Talkum eingestäubt und mit der Abdruckseite nach oben auf eine ebenfalls mit Talkum eingestäubte flache Platte übertragen. Die Strecke zwischen zwei parallellaufenden Linien im Abstand von 25 mm auf dem mit Linien versehenen Testblock wird unter einem Meßmikroskop auf 0,005 mm genau gemessen und als Meßwert A festgehalten. 24 Stunden nach Herstellung der Abformungen wird die Strecke zwischen den parallellaufenden Linien auf der Abformung gemessen und als Meßwert B festgehalten. Die Dimensionsveränderung errechnet sich aus $(A-B)/A \times 100$, der Durchschnitt aus 3 Bestimmungen.

Ergebnisse von den Messungen der Dimensionsänderung an den Abformmassen Beispiel 1-4 und Dubliermassen Beispiel 5-6 nach ADA-Spezifikation 19

| Basis/Katalysatorpaste Beispiel | | Änderung nach 24 Std. |
|---|---|---|
| 1 | 1 : 1 | −0,32 % |
| 2 | 1 : 1 | −0,14 % |
| 3 | 1 : 1 | −0,39 % |
| 4 | 1 : 1 | −0,16 % |
| 5 | 9 : 1 | −0,45 % |
| 6 | 9 : 1 | −0,19 % |

**Patentansprüche**

1. Dimensionsstabile, bei Umgebungstemperatur additionsvernetzende Abform- und Dubliermassen auf Polysiloxanbasis enthaltend.
   a) Organopolysiloxane mit zwei oder mehr Vinylgruppen im Molekül,
   b) gegebenenfalls Organopolysiloxane ohne reaktive Gruppen,
   c) organohydrogenpolysiloxane mit zwei oder mehr Si-H-Gruppen im Molekül,
   d) Katalysator,
   e) Füllstoffe sowie gegebenenfalls weitere übliche Zusatz-, Hilfs- und Farbstoffe,
dadurch gekennzeichnet, daß die flüssigen Polysiloxan-Komponenten a) - c) einen Gehalt an flüchtigen Oligomeren von maximal 1,5 Gew.- % aufweisen.

2. Massen gemäß Anspruch 1, dadurch gekennzeichnet, daß der Maximal Gehalt der flüchtigen Oligomere 0-0,8 Gew.- % beträgt.

3. Verfahren zur Herstellung von Abformungen, dadurch gekennzeichnet, daß man Massen gemäß einem der Ansprüche 1 oder 2 einsetzt.

4. Verwendung von Polysiloxanmassen gemäß einem der Ansprüche 1 oder 2 zur Herstellung von Abformungen im Dentalbereich.

**Claims**

1. Dimensionally stable impression compositions and duplicating compositions which undergo crosslinking at ambient temperature by an addition reaction, are based on polysiloxane and contain
   a) organopolysiloxanes possessing two or more vinyl groups in the molecule,
   b) if appropriate, organopolysiloxanes without reactive groups,
   c) organohydrogenopolysiloxanes possessing two or more Si-H groups in the molecule,
   d) a catalyst,
   e) fillers and, if appropriate, other customary additives, auxiliaries and colorants,
characterised in that the liquid polysiloxane components a) - c) contain not more than 1.5 % by weight of volatile oligomers.

2. Compositions according to Claim 1, characterised in that the maximum content of the volatile oligomers is 0-0.8 % by weight.

3. Process for the preparation of impressions, characterised in that compositions according to one of Claims 1 or 2 are employed.

4. Use of polysiloxane compositions according to one of Claims 1 or 2 for the preparation of impressions in the dental sector.

**Revendications**

1. Masses à mouler et à doubler à haute stabilité dimensionnelle, réticulantes par addition à température ambiante, à base de polysiloxanes, contenant
   a) des organopolysiloxanes avec deux ou plusieurs groupes vinyle dans la molécule,
   b) éventuellement des organopolysiloxanes sans groupes réactifs,
   c) des organohydrogénopolysiloxanes avec deux ou plusieurs groupes Si-H dans la molécule,
   d) un catalyseur,
   e) des agents de charge et éventuellement d'autres substances additionnelles, auxiliaires ou colorantes usuelles,

caractérisées en ce que les composants polysiloxanes liquides a) - c) présentent une teneur en oligomères volatils de 1,5 % en poids au maximum.

2. Masses selon la revendication 1, caractérisées en ce que la teneur maximale des oligomères volatils est de 0 à 0,8 % en poids.

3. Procédé de fabrication de pièces moulées, caractérisé en ce qu'on utilise des masses selon la revendication 1 ou 2.

4. Utilisation de masses de polysiloxane selon la revendication 1 ou 2 pour la fabrication de pièces moulées dans le secteur de la dentisterie.